# EUROPEAN PATENT APPLICATION

(11) **EP 2 394 669 A1**
(43) Date of publication of application: **14.12.2011**
(21) Application number: 10165680.9
(22) Date of filing: 11.06.2010
(51) Int. Cl.: A61L 15/22, A61L 15/28, A61L 15/60

(54) **Absorbent product comprising a cationic polysaccharide in a hydrophilic carrier matrix**

(71) Applicant: The Procter & Gamble Company, Cincinnati, OH 45202 (US)
(72) Inventor: Carlucci, Giovanni, 66100, Chieti (IT); Gagliardini, Alessandro, 65010, Pescara (IT); D'Alesio, Nicola, 66010, Chieti (IT)
(74) Representative: Briatore, Andrea

(57) **Abstract**

An absorbent product for feminine protection comprising a mixture of a cationic polysaccharide and a hydrophilic carrier matrix, wherein the cationic polysaccharide is a water-based cationic polysaccharide, and / or has a degree of cationization of less than 3% by weight.

## Description

### FIELD OF THE INVENTION

The invention relates to absorbent products for feminine protection, for example sanitary pads, pantiliners or tampons, comprising a cationic polysaccharide provided in a hydrophilic carrier matrix.

### BACKGROUND OF THE INVENTION

Most commercially available disposable absorbent products like sanitary napkins and diapers comprise synthetic superabsorbent polymers (SAP), typically polyacrylates, to deliver body fluid absorption and retention characteristics. Although such synthetic absorbent materials exhibit outstanding absorption capacity towards de-ionized water, their absorption capacity towards electrolytes/salts-containing solutions like menses is lower. It is assumed that the presence of electrolytes, proteins and cells (mainly red cells in menses) interfere with the swelling process of the absorbing gelling materials (see for ref. P.K. Chatterjee, B. S. Gupta, "Absorbent Technology" Elsevier 2002; pages 455-457).

Whereas synthetic superabsorbent polymers have been found to work very well to absorb fluids like urine, their performance can be disappointing in feminine care applications where at least part of the fluid to be absorbed is menstrual fluid. This can lead to the failure of the feminine care product to efficiently absorb the menstrual fluid and eventually leakage and soiling of the user's garments.

US 5,780,616 discloses cationic polysaccharides having superabsorbent characteristics. The polysaccharides are substituted by quaternary ammonium groups, having a degree of substitution of at least 0.5. The polysaccharide is preferably cellulose. The polysaccharides are cross-linked to a sufficient extent that they remain insoluble in water.

US 5,532,350 discloses crosslinked polysaccharides useful as absorbent material, among which guar gum and guar derivatives are typically used.

US 5,801,116 discloses crosslinked or not crosslinked polysaccharides, particularly guar polymers, such as for example guar gum, used as absorbent materials. The polysaccharides are typically water insoluble, or only slightly water soluble, wherein less than 50% of the polysaccharide dissolves in water.

US 6,887,564 to Procter & Gamble Company, discloses disposable absorbent products comprising chitosan material and an anionic absorbent gelling material. However, until now the high cost of chitosan materials has prevented their commercial uses.

In certain instances, for example in order to immobilize the absorbent material or to improve its processability, it may be desirable to provide the absorbent material in combination with a carrier matrix. WO 2007/017825 discloses various liquid absorbing materials comprising a particulate absorbent material having a particle size of less than 40µm, among these chitosan, which is dispersed in an inert hydrophilic organic carrier matrix.

However, with regard to the manufacture of commercial disposable absorbent products, such as sanitary napkins or panty liners, new developed materials, such as absorbent materials, need to meet certain process constraints. For example, processes often require that such materials can be remelted easily.

Accordingly, there is a general need to provide materials that can be easily handled during production processes. It is also desirable that the use of a new material does not add complicated steps to the manufacturing process or slows the process down.

In some production processes materials need to be transferred from a solid state in which they are stored into the melt before they can be applied to a product. It may therefore be desirable that such materials can be easily remelted.

### SUMMARY OF THE INVENTION

The invention relates to an absorbent product for feminine protection comprising a mixture of a cationic polysaccharide and a hydrophilic carrier matrix, wherein the cationic polysaccharide
- is a water-based cationic polysaccharide; and / or
- has a degree of cationization of less than 3% by weight, or less than 2% by weight, or even less than 1% by weight.

The invention further relates to a process for producing a product for feminine protection comprising the steps of
- providing a cationic polysaccharide in form of a powder,
- providing a hydrophilic carrier matrix,
- melting the hydrophilic carrier matrix,
- mixing the powder of cationic polysaccharide with the molten carrier matrix to form a mixture of the cationic polysaccharide and the hydrophilic carrier matrix,
- applying the mixture of the cationic polysaccharide and the hydrophilic carrier matrix to at least a part of a product for feminine protection;
   wherein the cationic polysaccharide
- is a water-based cationic cationic polysaccharide; and / or
- has a degree of cationization of less than 3% by weight, or less than 2% by weight, or even less than 1% by weight.

The present inventors found that cationic polysaccharides having a low degree of cationization, such as water-based cationic polysaccharide and / or cationic polysaccharides having a degree of cationization of less than 3% by weight when provided in a hydrophilic carrier matrix show advantageous melting properties. For example, mixtures comprising a cationic polysaccharide and a hydrophilic carrier matrix have been found to remelt better compared to mixtures comprising cationic polysaccharides which are not water-based, and / or do not have a higher degree of cationization.

### DETAILED DESCRIPTION OF THE INVENTION

The term "absorbent product for feminine protection" refers to products normally used by women for absorbing menses as well as adult light to moderate incontinence products. These products are usually disposable, i.e. are discarded after usage. Absorbent products for feminine protection include menses absorbing products such as sanitary napkins, panty liners, tampons, and interlabial pads.

The term "disposable" is used herein to describe products, which are not intended to be laundered or otherwise restored or reused as a product (i.e. they are intended to be discarded after a single use and possibly to be recycled, composted or otherwise disposed of in an environmentally compatible manner).

"Cationic polysaccharide" as used herein refers to the product of the reaction between a polysaccharide and a suitable cationizing agent.

The term "cationic starch" as used herein refers to the product of the reaction between starch and a suitable cationizing agent. Usually, cationic starches may have a net positive charge in aqueous solutions at a pH range from 3 to 10, in particular between pH of 5 and 9.

"Cationic guar gum" as used herein refers to the product of the reaction between guar gum and a suitable cationizing agent. Usually, cationic guar gums may have a net positive charge in auqueous solutions at a pH range from 3 to 10, in particular between a pH range from 5 to 9.

"Water-based cationic polysaccharides" are obtained by performing the cationization reaction on the polysaccharide using water as reaction media. The water-based cationic polysaccharides may for example be synthesized according to US 2,813,093.

The cationic polysaccharides described herein have been modified with a nitrogen containing cationization agent and the "degree of cationization" as used herein refers to the nitrogen content of the cationic polysaccharide. The degree of cationization herein is given as the nitrogen content in weight percent (wt%) relative to the total weight of the cationic polysaccharide.

"Absorbent material" as used herein refers to materials which swell upon contact with water containing liquids, such as menses, blood or urine. Typically, absorbent materials are able to absorb and hold liquids in a magnitude of several times their own weight. Exemplary conventional absorbent materials are polyacrylate based gelling materials. Among the conventional absorbent materials are also superabsorbent materials, which for example absorb liquids in the magnitude of more than hundred times their own weight.

The absorbent products for feminine protection described herein comprise a cationic polysaccharide and a hydrophilic carrier which are mixed, so that the hydrophilic carrier forms a matrix in which the cationic polysaccharide is included. This mixture is referred to herein as "mixture of cationic polysaccharide and hydrophilic carrier matrix".

The cationic polysaccharide may be used to absorb and / or immobilize menses. When the cationic polysaccharide is used as an absorbent material, it may be used as sole absorbent material, or alternatively may be used in combination with other absorbent materials, such as conventional superabsorbent polymers.

The hydrophilic carrier matrix is used in order to enhance the contact with water containing liquids, such as blood, menses or urine. The hydrophilic carrier matrix acquires and diffuses the liquid and rapidly brings it in contact with the cationic polysaccharide dispersed therein, which in turn can perform its liquid absorbing and / or liquid immobilizing action with enhanced effectiveness.

A mixture of cationic polysaccharide and hydrophilic carrier matrix may for example be formed by melting the hydrophilic carrier matrix, adding the cationic polysaccharide and mixing both by suitable means.

The cationic polysaccharide may be added in the form of a powder comprising particles having a size of 5 µm ― 80 µm, or of 10 µm ― 60 µm, for example of 20 to 50 µm

In its solid state, the mixture of cationic polysaccharide and hydrophilic carrier matrix may be stored for longer periods of time before being incorporated into an absorbent product for feminine protection.

During subsequent production steps and processes, it may be necessary to remelt the mixture of cationic polysaccharide and hydrophilic carrier matrix in order to apply it to the absorbent product for feminine protection. Thus, it is desirable that, after storing, the mixture of cationic polysaccharide and hydrophilic carrier matrix can be easily transferred from its solid state to the melt.

It has been found by the inventors that the degree of cationization of the polysaccharide can influence the melting properties of the mixture of cationic polysaccharide and hydrophilic carrier matrix.

Mixtures comprising cationic polysaccharides having a lower degree of cationization in a hydrophilic carrier matrix have for example been observed to have melting properties that allow a fast and efficient incorporation into the absorbent products. A low degree of cationization, such as less than 3% by weight, or less than 2% by weight, or even less than 1% by weight, has for example been found to promote the remelting ability of mixtures of cationic polysaccharides and hydrophilic carrier matrix. An improved remelting ability can for example be seen in a reduction in the time needed to melt a given amount of the mixture.

Cationic polysaccharides having a degree of cationization of less than 3% by weight, or less than 2% by weight, or even less than 1% by weight as well as water-based cationic polysaccharides which typically have a degree of cationization of less than 1%, or even less than 0.8% by weight have been found particularly suitable. Typically, cationic polysaccharides have a degree of cationization of more than 0.001 %, or even more than 0.01 % by weight.

### Absorbent product for feminine protection

An absorbent product for feminine protection may comprise an absorbent core. "Absorbent core" as used herein refers to a member of an absorbent product that is intended to absorb and store exudates, such as menses, discharged from the body. Thus, the absorbent core typically comprises an absorbent material, such as conventional synthetic superabsorbent polymers and / or the mixtures of cationic polysaccharides and hydrophilic carrier matrix described herein.

The absorbent core may be of any suitable shape. For example rectangular, ellipsoid or hourglass like.

For purposes of illustration an absorbent core may be described as having a longitudinal axis defining its length and orthogonal thereto a transversal axis defining its width, which will typically be smaller than its length. The center of the absorbent core typically corresponds to its center of mass.

An absorbent core used in a product for feminine protection, may comprise a fluff matrix, also referred to as "airfelt", of cellulose pulp, or a mixture of cellulose pulp with synthetic fibers.

Thin cores such as those disclosed for example in EP1447067 may also be used. In certain embodiments, the absorbent core may only comprise comparably low amount of airfelt, for example less than 30%, or even less than 10% by weight of the entire absorbent core may comprise airfelt material. In other embodiments, the absorbent core may even be free of airfelt.

The core may also comprise conventional synthetic superabsorbent materials such as polyacrylate based gelling materials. Optionally, the absorbent core may comprise an anionic absorbent gelling material.

The mixture of cationic polysaccharide and hydrophilic carrier matrix may be applied on at least a part of the absorbent core.

It has further been found that the mixture of hydrophilic carrier matrix and cationic polysaccharide having a degree of cationization of less than 3%, or 2%, or even 1% by weight can improve the immobilization of menses.

The mixture of cationic polysaccharide and hydrophilic carrier matrix may be used as a barrier to the lateral movement of menses in order to avoid or reduce leakage and soiling of the wearer's clothes.

Thus, the mixture of cationic polysaccharide and hydrophilic carrier matrix may be applied to the outer periphery of the absorbent core, for example in the proximity of the outer edges of the absorbent core.

The basis weight (mass per unit area) of the mixture of cationic polysaccharide and hydrophilic carrier matrix may gradually or continuously increase from the center of the absorbent core towards its outer periphery.

In order to provide fast acquisition and distribution of liquids at the point of insult, the center of the absorbent core may be free of the mixture of cationic polysaccharide and hydrophilic carrier matrix. For example, an area of at least 2 cm², or at least 5 cm² comprising the center of the absorbent core, may be free of the mixture of cationic polysaccharide and hydrophilic carrier matrix.

The mixture of cationic polysaccharide and hydrophilic carrier matrix may be applied to the absorbent core in stripes. The stripes may be substantially parallel to the longitudinal axis of the absorbent core.

The absorbent core may comprise at least two stripes of cationic polysaccharide and hydrophilic carrier matrix wherein the stripes may be laterally spaced from the center of the absorbent core by a distance. For example, the stripes may be spaced apart from the center by a distance of at least 1 cm, or at least 2 cm.

The stripes may have a width substantially parallel to the lateral axis of the absorbent core of more than 0.2 cm, or more than 0.5 cm, or even more than 1cm and less than 3 cm or even less than 2 cm.

### Mixture of cationic polysaccharide and hydrophilic carrier matrix

In order to effectively absorb and / or immobilize menses, it has been found advantageous that the mixture of cationic polysaccharide and hydrophilic carrier matrix comprises high amounts of the polysaccharide, for example at least 30%, such as between 30 and 40% by weight of the mixture of cationic polysaccharide and hydrophilic carrier matrix. The mixture of cationic polysaccharide and hydrophilic carrier matrix may even comprise at least 40% or at least 50% cationic polysaccharide by weight of the mixture of cationic polysaccharide and hydrophilic carrier matrix. Typically, the mixture of cationic polysaccharide and hydrophilic carrier matrix may not comprise more than 80 wt% cationic polysaccharide, or not more than 70 wt% of cationic polysaccharide.

With regard to mixtures comprising high amounts of cationic polysaccharide, a lower degree of cationization of the cationic polysaccharide has been found advantageous. For example mixtures comprising at least 30% cationic polysaccharide (by weight of the mixture of cationic polysaccharide and hydrophilic carrier matrix), cationic polysaccharides having a degree of cationization of below 3wt%, or below 2wt% or even below 1wt% have been found to have a better processability, for example due to shorter remelting times, when compared to polysaccharides having a degree of cationization of about 3 wt% or higher.

For the application in an absorbent product for feminine protection, it may be advantageous that the mixture of cationic polysaccharide and hydrophilic carrier matrix is solid at room temperature and at body temperatures. For example, the mixture of cationic polysaccharide and hydrophilic carrier matrix may be solid at temperatures below 40°C, or even below 45°C.

On the other hand, a relatively low melting temperature may be advantageous when a production process requires remelting of the mixture of cationic polysaccharide and hydrophilic carrier matrix. Thus, in certain embodiments, the mixture of cationic polysaccharide and hydrophilic carrier matrix may melt at temperatures below 100°C or 80°C or even 70°C.

### Cationic polysaccharides

Cationic polysaccharides for use in the invention are polysaccharides and polysaccharide derivatives that have been cationized by chemical means, e.g. quaternization with quaternary amine compounds containing reactive chloride or epoxide sites. The chemical compounds used for cationization will also be referred to as "cationic agents" or "cationization agents".

Cationic polysaccharides can be obtained by methods well known to a person skilled in the art. For example, the cationic polysaccharides may be synthesized according to US 2,813,093.

Cationic polysaccharides for use in the invention may be cationic guar gum and / or cationic starch.

The source of starch used for cationic modification can be chosen from any usual sources including tubers, legumes, cereal, and grains. Non-limiting examples of starch may include corn starch, wheat starch, rice starch, waxy corn starch, oat starch, cassaya starch, waxy barley, tapioca starch, potato starch or mixture thereof. Starch, particularly native starch, comprises polymers made of glucose units. There are two distinct polymer types. One type of polymer is amylose whereas the other is amylopectin. In one embodiment, the cationic starch of the present invention may comprise a starch comprising amylopectin at a level of from about 90-100% Wt and more specifically above 95% Wt. Various methods for making cationic modified starches are known in the art, see for example these disclosed in US2,813.093 and US4,281,109. The cationic modified starches used in the present invention can be easily made by a skilled person using these known chemical reactions. For example, a waxy type starch may be used and reacted with 2,3 epoxypropyl-N, N, N-trimethyl ammonium chloride (commercially available as Quab 151) as cationizing agent.

The source of guar gum for cationic modification is typically the guar bean. Guar gum, also known as guar flour, comprises high molecular weight polysaccharides composed of galactomannans. The water soluble fraction of guar gum is called guaran and typically consists of linear chains of (1→6)-β-D-mannopyranosyl units (D-mannose) with α-D-galactopyranosyl units (D-galactose) attached by (1→6) linkages. Ratio of D-galactose to D-mannose is 1:2. Methods for providing guar gums with cationic functionality are known in the art, for example as disclosed in US 2008/0112907, which describes water dispersible polygalactomannan polymers.

The cationic modified polysaccharides used in the invention have a cationization degree of less than 3 wt%. The cationization degree may be less than 2 wt%, or even less than 1 wt%.

Thus, the cationic polysaccharide for use in the present invention may be a water based cationic polysaccharide. A water-based cationic polysaccharide, such as a water-based cationic starch, is obtained by performing the cationization reaction using water as reaction media. For practical reasons, the so obtained water-based cationic polysaccharides have typically a low degree of cationization. For example, the degree of cationization of water-based cationic polysaccharides may be less than 1.0 wt%, for example less than 0.8 wt%.

The cationic agents used in the fabrication of the cationic polysaccharides according to the present invention can typically comprise an ammonium group.

Suitable cationic agents comprising an ammonium group include for example those listed in US5,780,616 col.4 line 5 to col.5 line 15. In particular the following examples:
- glycidyltrimethylammonium chloride;
- 2,3-epoxypropyl-N,N,N-trimethylammonium chloride (commercially available from Degussa A. G. as a 70% aqueous solution under the name QUAB 151 or as the pure compound in solid form from Fluka under product code 50045) having for structural formula: 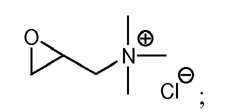
- 3-chloro-2-hydroxypropyl-N,N,N-trimethylammonium chloride (CAS # 3327-22-8, commercially available from Degussa A. G. as a 65% aqueous solution under the name of QUAB 188), having the structural formula: 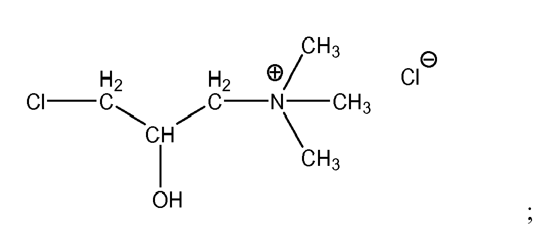
- 3-chloro-2-hydroxypropyl-N,N,N-dimethylethanolammonium chloride (commercially available from Degussa A. G. as a 65% aqueous solution under the name of QUAB 218), ("DEC", CAS # 869-24-9), having the structural formula: 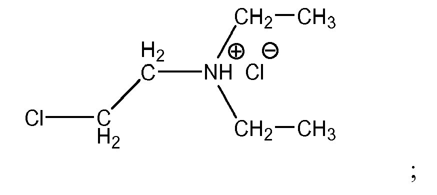
- 1,3-bis-(3-chloro-2-hydroxypropyl-N,N-dimethylammonium)-N-propane dichloride (commercially available from Degussa A. G. as a 65% aqueous solution under the name of QUAB 388);

Particularly advantageous quaternary ammonium compounds among these are 2,3-epoxypropyltrimethyl ammonium chloride, N-(3-chloro-2-hydroxypropyl) trimethyl ammonium chloride, and diethylaminoethylchloride hydrochloride.

### Crosslinked cationic polysaccharides

The cationic polysaccharides may be crosslinked.

Methods for cross-linking polysaccharides, such as starch or guar gum, with and without cationic modification are for example disclosed in US5,532,350, US5,801,116, US2008/0112907, US5,780,616 and WO92/19652.

The level of cross-linking of a cationic polysaccharide and hence the solubility of the cationic polysaccharide can be controlled by the skilled person during the synthesis, in particular the concentration of cross-linking agents in the reaction mixture can be varied to obtain the desired amount of cross-linking.

It was found that a concentration of cross-linking agent of from about 100 ppm to about 4000 ppm (parts per million) in the reaction mixture may be advantageous to obtain the desired amount of cross-linking. More particular ranges are from about 150 ppm to 3500 ppm, and from about 200 ppm to 3000ppm. By "ppm" we mean the relative amount of the cross-linking agent expressed in weight units per weight of the starch material to be cross-linked expressed in parts per million.

The amount of cross-linking can also be expressed by reference to the degree of substitution of the cationic polysaccharide by the cross-linking agents (herein referred to as "degree of substitution of the cross-linking agent"), which may advantageously be less than 0.0010, for example from about 0.00005 to about 0.00095, or from about 0.00010 to about 0.00080. The degree of substitution of the cross-linking agent is sometimes used in the literature (see GB1,576,475 and US3,622,562 for example).

Suitable cross-linking agents include for example:
- formaldehyde;
- methylolated nitrogen compounds such as dimethylolurea, dimethylolethyleneurea and dimethylolimidazolidone;
- diacarboxylic acids such a maleic acid;
- dialdehydes such as glyoxal;
- diepoxides such a 1,2:3,4-diepoxybutane and 1,2:5,6-diepoxyhexane;
- diisocyanates;
- divinyl compounds such as divinylsulphone;
- dihalogen compounds such as dichloroacetone, dichloroacetic acid, 1,3-dichloropropan-2-ol, dichloroethane, 2,3-dibromo-1-propanol, 2,3-dichloro-1-propanol and 2,2-dichloroethyl ether;
- halohydrins such as epichlorohydrine;
- bis(epoxypropyl)ether;
- vinylcyclohexenedioxide;
- ethylene glycol-bis(epoxypropyl)ether;
- 1,3-bis(β-hydroxy-Γ-chloropropoxy)-2-propanol;
- 1,3-bis(β-hydroxy-Γ-chloropropoxy)ethane;
- methylenebis(acrylamide);
- N,N'-dimethylol(methylenebis(acrylamide));
- triacrylolhexahydrotriazine;
- acrylamidomethylene chloroacetamide;
- 2,4,6-trichloropyrimidine;
- 2,4,5,6-tetrachloropyrimidine
- cyanuric chloride;
- triallylcyanurate
- phosphorusoxychloride;
- bis(acrylamido)acetic acid.

In particular, epichlorohydrine ("EPI") and phosphorusoxychloride ("POC13") are commonly used in this type of reactions and may be considered advantageous.

### Hydrophilic carrier matrix

The hydrophilic carrier matrix may comprise one or more polymers selected from the group consisting of polyglycols, polyvinylalcohols, ethoxylated surfactants and mixtures thereof.

In order to have a good processability, the polymers of the hydrophilic carrier matrix may be selected according to their molecular weight and / or melting temperatures.

Thus, the polymers comprised by the hydrophilic carrier matrix may have a weight average of molecular weight of 500 - 35000 g/mol, preferably of 1000 - 3500 g/mol.

The hydrophilic carrier matrix can for example comprise polyethylene glycols, polypropylene glycols, and derivatives thereof, such as for example polyoxymethylene glycols. Polyethylene glycols, polypropylene glycols, and derivatives thereof are moreover highly hydrophilic, and hence have a good affinity with water and water based liquids.

In certain embodiments, the hydrophilic carrier matrix may melt at a temperature in the range of from 30°C to 100°C. For example, the hydrophilic carrier matrix may melt at a temperature in the range from 25°C to 80°C.

The hydrophilic carrier matrix may be comprised of polyethylene glycol (PEG). The PEG may for example have a weight average of molecular weight (MW) of 1000 - 10000 g/mol, or of 1000 - 3500 g/mol, or even 1200 - 2000 g/mol.

### Process for producing a product for feminine protection

It is envisaged that the mixture of cationic polysaccharide and hydrophilic carrier matrix may be advantageously applied to at least a part of an absorbent product for feminine protection. For example, the mixture may be applied to a part of the absorbent core before this component is assembled with the other components forming the product.

However, in an alternative embodiment, the mixture may be applied to at least a part of the finished product.

The invention thus also relates to a process for producing a product for feminine protection, comprising the steps of
- providing a cationic polysaccharide in form of a powder;
- providing a hydrophilic carrier matrix;
- melting the hydrophilic carrier matrix and mixing the powder of cationic polysaccharide with the molten hydrophilic carrier matrix to form a mixture of cationic polysaccharide and hydrophilic carrier matrix,
- applying the mixture of cationic polysaccharide and hydrophilic carrier matrix to at least a part of the product of feminine protection;
wherein the cationic polysaccharide
- is a water-based cationic polysaccharide; and / or
- has a degree of cationization of less than 3% by weight, or less than 2% by weight, or even less than 1% by weight.

The cationic polysaccharide can be mixed with the hydrophilic carrier matrix by using conventional methods such as batch mixing or continuous extrusion.

In a batch mixing process for example, the hydrophilic carrier matrix may be placed in a vessel, mechanically stirred and heated to the melt. After melting of the hydrophilic carrier matrix the cationic polysaccharide may be added in the form of a powder. Stirring is continued until a homogeneous mixture is obtained, which may be further processed, or disposed into containers, allowed to cool and stored until further use.

The mixture of cationic polysaccharide and hydrophilic carrier matrix may be processed in the form of a melt in a temperature range between 50°C - 80°C.

The mixture of cationic polysaccharide and hydrophilic carrier matrix may be allowed to cool and solidify. Subsequently, it may be remelted and then applied to the product for feminine protection.

The molten mixture of cationic polysaccharide and hydrophilic carrier matrix may then be applied to at least a part of the product for feminine protection. For example, the mixture may be applied to the absorbent core in the form of stripes.

### METHODS / MEASUREMENTS

### Degree of cationization

The degree of cationization (i.e. nitrogen content in wt%) can be determined by elemental analysis.

The degree of cationization may for example be determined using a nitrogen analyzer, for example a LECO FP-2000 nitrogen analyzer. This instrument is typically used to determine the total nitrogen content in solid materials. It operates on the Dumas method, where the sample is combusted at 850+ °C in an oxygen rich atmosphere. Combustion gases are collected in the 4.5-liter volume ballast after passing through a thermoelectric cooler to remove H₂O. A 10 mL portion of the combustion gases are scrubbed of water and carbon dioxide and passed through a hot copper column. The resulting nitrogen gas is then measured by thermal conductivity in a helium carrier. Each sample analysis is completed in about 4 minutes.

### Particle size

Particle size can be measured by using a Beckman Coulter LS 1320 particle analyzer according to the ISO standard covering particle sizing by the laser diffraction method (ISO/DIN 13320-1 Particle size analysis -- Laser diffraction methods -- Part 1: General principles).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent product for feminine protection comprising a mixture of a cationic polysaccharide and a hydrophilic carrier matrix, **characterized in that** the cationic polysaccharide has been modified by a nitrogen containing cationization agent and
- is a water-based cationic polysaccharide; and / or
- has a degree of cationization of less than 3% by weight, preferably less than 2% by weight, more preferably less than 1% by weight.

2. The absorbent product for feminine protection of claim 1, wherein the cationic polysaccharide is a cationic starch or a cationic guar gum.

3. The absorbent product for feminine protection of any of the preceding claims, wherein the cationic polysaccharide is provided in the form of particles having an average particle size of from 5 µm to 80 µm, preferably from 10 to 60 µm and more preferably from 20 to 50 µm

4. The absorbent product for feminine protection of any of the preceding claims, wherein the cationic polysaccharide is a derivative of a starch selected from the group consisting of corn starch, wheat starch, rice starch, waxy corn starch, oat starch, cassaya starch, waxy barley, tapioca starch, potato starch or mixtures thereof.

5. The absorbent product for feminine protection of any of the preceding claims, wherein the cationic polysaccharide is crosslinked.

6. The absorbent product for feminine protection of any of the preceding claims, wherein the hydrophilic carrier matrix comprises one or more polymers selected from the group consisting of polyglycols, polyvinylalcohols and ethoxylated surfactants and mixtures thereof.

7. The absorbent product for feminine protection of claim 6, wherein the one or more polymers comprised by the hydrophilic carrier matrix have a weight average of molecular weight of from 1000 to 10000 g/mol.

8. The absorbent product of claims 6 or 7, wherein the hydrophilic carrier matrix comprises polyethyleneglycol.

9. The absorbent product for feminine protection of any of the preceding claims, comprising at least 15%, preferably at least 30% cationic polysaccharide by weight of the mixture of cationic polysaccharide and hydrophilic carrier matrix.

10. The absorbent product for feminine protection of any of the preceding claims, wherein the hydrophilic carrier matrix melts at a temperature in the range of from 30°C to 100°C.

11. The absorbent product for feminine protection of any of the preceding claims, wherein the product for feminine protection is selected from the group consisting of a sanitary napkins, pantiliners, tampons and interlabial pads.

12. The absorbent product for feminine protection of any of the preceding claims further comprising an absorbent core, wherein the mixture of cationic polysaccharide and hydrophilic carrier matrix is applied on at least a part of the absorbent core.

13. The absorbent product for feminine protection of claim 12, wherein the mixture of cationic polysaccharide and hydrophilic carrier matrix is applied in stripes.

14. A process for producing a product for feminine protection comprising the steps of
- providing a cationic polysaccharide in form of a powder,
- providing a hydrophilic carrier matrix,
- melting the hydrophilic carrier matrix,
- mixing the powder of cationic polysaccharide with the molten carrier matrix to form a mixture of the cationic polysaccharide and the hydrophilic carrier matrix,
- applying the mixture of cationic polysaccharide and hydrophilic carrier matrix to at least a part of a product for feminine protection;
**characterized in that** the cationic polysaccharide has been modified by a nitrogen containing cationization agent and
- is a water-based cationic polysaccharide; and / or
- has a degree of cationization of less than 3% by weight.

15. The process of claim 14, wherein the mixture of cationic polysaccharide and hydrophilic carrier matrix is allowed to cool and solidify and then remelted before being applied to the product for feminine protection.
